# EUROPÄISCHE PATENTANMELDUNG

(11) **EP 2 221 045 A1**
(43) Veröffentlichungstag der Anmeldung: **25.08.2010**
(21) Anmeldenummer: 10000951.3
(22) Anmeldetag: 30.01.2010
(51) Int. Cl.: A61K 8/81, A61K 8/84, A61Q 19/00

(54) **Hautpflege-Formulierungen mit einem sofort spürbaren Straffungseffekt**

(30) Priorität: 20.02.2009 DE 102009009758
(71) Anmelder: Beiersdorf AG, 20245 Hamburg (DE)
(72) Erfinder: Heuer, Björn, 22145 Hamburg (DE); Kröpke, Rainer, 22869 Hamburg (DE); Hiddemann, Sarah, 20251 Hamburg (DE); Kummer, Andreas B., 21079 Hamburg (DE); Fänger, Sabine, 22763 Hamburg (DE)
(74) Vertreter: Hartmann, Jost

(57) **Zusammenfassung**

Die Erfindung umfasst die Verwendung von mindestens einem polymeren Filmbildner, der ein oder mehrere kationische und/oder kationogene Monomere enthält, und mindestens einem polymeren anionischen Verdicker in kosmetischen oder dermatologischen Zubereitungen zur Hautstraffung. Die Zubereitungen zeigen einen sofort spürbaren Straffungseffekt.

## Beschreibung

Die Erfindung umfasst die Verwendung einer Kombination von mindestens einem polymeren Filmbildner, der ein oder mehrere kationische und/oder kationogene Monomere enthält, und mindestens einem polymeren anionischen Verdicker in kosmetischen Zubereitungen zur Hautstraffung.
Die erfindungsgemäßen kosmetische oder dermatologische Zubereitungen zeigen nach Hautapplikation einen sofort spürbaren Straffungseffekt.

Hautpflegeprodukte, die eine Wirkung gegen Falten aufweisen, werden im Kosmetik-Markt immer beliebter. Nachteilig aus Sicht der Verbraucher ist häufig, dass die Wirkung durch die Inhaltsstoffe, die Falten bzw. Faltenbildung reduziert, kaum wahrnehmbar ist, da es sich bei der Faltenreduktion um Langzeiteffekte handelt. Es ist daher üblich, durch spezielle sensorische Reize zu versuchen, die Wirkung der Produkte erlebbarer zu machen.

Wünschenswert ist es neben der Faltenreduktion einen hautstraffenden Effekt zu erzielen.

Es finden sich in der Literatur, bei Rohstoffinformationen von Polymerherstellern, sowie in zahlreichen Patenten viele Beispiele, wie dieser Effekt mit Hilfe von Polymeren zu erreichen sein könnte.
Grundlage des Straffungseffektes durch Polymere ist die Tatsache, dass sich Polymerfilme bei der Bildung aus einer Lösung häufig leicht zusammen ziehen. Bei einer entsprechenden Haftung auf der Haut ist dabei eine Straffung zu spüren. Dieser Effekt ist vergleichbar mit der Applikation von selbstklebenden Pflastern auf der Haut, wie beispielsweise bei der Wundbehandlung und der Vermeidung der Bildung unschöner Narben.

Dieses Wirkprinzip zeigt aber auch einige Nachteile:
1. Die Wirkung wird durch weitere Formelbestandteile in der Zubereitung, die nicht flüchtig sind und daher beim Trocknen zusammen mit dem Polymer auf der Haut verbleiben, stark gestört, wie insbesondere Fette, Öle, Emulgatoren etc. Daher ist in der Regel eine leicht angedickte wässrige Lösung möglich; Dispersionen und Emulsionen zeigen entsprechende Straffungseffekte jedoch kaum.
2. Wasserlösliche Bestandteile (z.B. Glycerin) sorgen oft für die Ausbildung eines sehr stark klebrigen Films auf der Haut, der als unangenehm empfunden wird.
3. Der ausgebildete Polymerfilm hat häufig eine sehr geringe kosmetische Anmutung und wirkt sehr künstlich auf der Haut (wie eine Maske).

Zahlreiche Patentanmeldung versuchen mit speziellen Polymeren, insbesondere Polymere mit besonderer Mikrostruktur (Pfropfpolymere, Sternpolymere, Dendrimere) einen solchen Straffungseffekt zu erreichen. Zu der Tatsache, dass solche Polymere durch den hohen Syntheseaufwand schwer zugänglich und teuer sind, ist zudem eine hautstraffende Wirkung nicht immer gegeben.

So wird in den Dokumenten des Standes der Technik der Straffungseffekt beschrieben durch

| | |
|---|---|
| EP 1419763 | Kombination von Polymerpartikeln mit amphiphilen Polymeren, |
| EP 0987016 | Kombination von filmbildenden Polymeren mit dendrimeren Polyestern, |
| US 2002/131948 | Silikon-Pfropf-Polymere, |
| EP 1038519 | Silikon-Pfropf-Polymere, |
| WO 9829092 | Dispersionen von Polymeren mit hoher Molmasse und einem speziellen mechanischen Profil, |
| FR 2821744 | dispergierte Polymerpartikel in einer Fettphase, |
| FR 2863494 | Dispersion von Pfropfpolymer-Partikeln in einer Fettphase, |
| FR 2860157 | nicht elastische, nicht wasserlösliche lineare Blockpolymere, |
| EP 1566171 | spezielles Tetrapolymer (Methacrylsäure, Methylmethacrylat, Butylacrylat und C16-C20 Alkylacrylat), |
| EP 1865918 | Styrol/Ethylacrylat Copolymere, |
| US 6630131 | eine Formulierung, enthaltend ein Silikat, ein Polysaccharid und ein AMPS-Polymer, |
| EP 1824447 | synthetische Polymere in Kombination mit Hydroxyalkylharnstoff, |

Firmenbroschüre der Firma interpolymer zu Syntran PC 5100, in der Kombinationen aus Polyacrylate-21 /Acrylates/Dimethylaminoethyl Methacrylate Copolymer, Acrylates/Ceteth-20 Itaconate Copolymer, Hydroxypropyl Starch Phosphate,Acrylates / C10-30 Alkyl Acrylates Crosspolymer und PC 5100 und Xanthan Gum beschrieben werden.

J. Jachowicz et. al., Cosmetic Science Technology, 2005, 206-215 ("Skin Tightening with Polymer-Containing Formulations")

T. Martins et. al., Happi, 2007, 44, 7, 68-71 ("Fast Acting, Skin Firming Polymer Technology")

COSMETICS & TOILETRIES, 2007, 122, 9, 86 (Sesaflash - Seppic Rohstoff enthaltend Polymere zur Hautstraffung)

COSMETICS & TOILETRIES, 2007, 122, 8, 105 (Quicklift - BASF Rohstoff mit Straffungseffekt)

COSMETICS & TOILETRIES, 2007, 122, 2, 82 (Laracare A200 - Lonza Polysaccharid mit Straffungseffekt)

DE 10 2006 005 451 A1 offenbart Formulierungen mit kationischen Polymeren (z.B. Polyquaternium-16) mit anionischen Verdickern (z.B. Carbomer) zur Verwendung als Styling-Produkt. Der Aspekt der Applikation auf die Haut, bzw. die Beschreibung eines Straffungseffekts findet sich hier nicht.

DE 10 2005 014 423 A1 offenbart Formulierungen mit kationischen Polymeren mit anionischen Verdickern (bevorzugt Carbopol Aqua SF-1) zur Verwendung in Shampoos und Waschgelen. Auch hier ist eine Hautpflegeanwendung oder der Aspekt der Hautstraffung nicht erwähnt.

DE 10 2004 062771 A1 offenbart Formulierungen mit anionischen Verdickern und Partikeln, die ebenfalls kationische Conditioner-Polymere enthalten können. Die Anwendung ist auf Körperreinigung beschränkt, wobei auch ein hautpflegender Aspekt beschrieben wird, der bekanntermaßen durch die Wahl der Partikel und verwendeten Ölkomponenten gewonnen wird.

DE 19926167 A1 offenbart Stylingmittel (Schaumfestiger und Gele), enthaltend Ubichinone (Q10) zum Schutz von Haar und Kopfhaut durch Oxidationsprozesse. Die Aufzählung der für solche Formulierungen möglichen Zusatzstoffe beinhaltet auch kationische und anionische Polymere.

Wünschenswert ist es eine Zubereitung bereit zu stellen, die mit einfachen, kosmetisch zugelassenen Bestandteilen Anti-Falten Produkte mit sofort spürbarer Hautstraffung ermöglichen, die die Nachteile des Standes der Technik nicht aufweisen.

Der Stand der Technik konnte dabei den Weg zur vorliegenden Erfindung jedoch nicht weisen.

Die Erfindung umfasst die Verwendung von mindestens einem polymeren Filmbildner, der ein oder mehrere kationische und/oder kationogene Monomere enthält, und mindestens einem polymeren anionischen Verdicker in kosmetischen Zubereitungen zur Hautstraffung.
Bevorzugte Zubereitungen liegen als wässrige Gelen, Hydrodispersionen oder O/W Emulsionen vor.
Unter Hautstraffung wird, wie das Wort "straffen" definiert, ein spürbarer Druck/Zug auf der Haut umfasst. Die Folge ist u.a. die Verbesserung des äußeren Erscheinungsbildes bei Cellulite, Verbesserung oder Verminderung von Hautunebenheiten oder Falten.

Völlig überraschend führt die Kombination kationischer bzw. kationogener Monomere mit anionischen polymeren Verdickern in kosmetischen Zubereitungen und angewendet auf der Haut zu einem sofort spürbaren Straffungseffekt auf der Haut

Besonders unerwartet ist der Effekt durch die kationischen bzw. kationogenen Polymere daher, da solche Verbindungen in der Hautpflege bislang praktisch nicht eingesetzt werden, sondern nur aus der Haarpflege bekannt sind. Zudem erwartet der Fachmann bei der Kombination solcher filmbildenden Polymere mit anionischen Verdickern Unverträglichkeiten, wie Ausfällungen von Polymerkomplexen, die auf die unterschiedlichen Ladungen zurückzuführen sind.
Diese Nachteile zeigen sich überraschenderweise jedoch nicht, so dass die Verwendung der erfindungsgemäßen Zubereitung als Hautstraffungsmittel einen vorteilhaften Beitrag in der Kosmetik leisten kann.

Die erfindungsgemäßen polymeren Filmbildner sind Polymere, die ein oder mehrere kationische und/oder kationogene Monomere enthalten. Als kationische Monomere sind Monomere erfindungsgemäß, die eine kationische Ladung tragen. Bevorzugt ist diese Ladung im Bereich von pH 2 bis pH 12 unabhängig vom pH-Wert.
Als kationogene Monomere sind Monomere zu verstehen, die eine oder mehrere protonierbare Gruppen haben, die abhängig vom pH-Wert protoniert und damit kationisch vorliegen.

Bevorzugt sind die kationische und/oder kationogene filmbildende Polymere wasserlöslich.
Bevorzugt werden hierbei Polymere auf Basis von Acryl- oder Methacrylsäureestern verwendet, wobei die Acryl- oder Methacrylsäureester mit einer Aminogruppe subsituiert ist, welche gegebenenfalls alkyliert (quaternisiert) ist. Es ist dabei unerheblich, ob das Polymer zusätzlich zu den kationischen bzw. kationogenen Monomeren nur nichtionische oder aber auch noch anionische bzw. anionogene Monomere enthält.
Als vorteilhafte Beispiele seien Dimethylaminoethylmethacrylat und Dimethylaminopropylmethacrylamid sowie deren methylierte oder ethylierte Analoga erwähnt. Bevorzugt eingesetzte polymeren Filmbildner dieser Art sind
Vinylcaprolactam/VP/Dimethylaminoethylmethacrylate Copolymer (CAS: 2-Propenoic acid, 2-methyl-, 2-(dimethylamino)ethyl ester, polymer with 1- ethenylhexahydro-2H-azepin-2-one and vinylpyrrolidone) z.B. Advantage® Typen von ISP, Polyquaternium-11, z.B. Gafquat® Typen von ISP oder Luviquat® PQ 11 PN von BASF, Polyquaternium-89, z.B. Ultrahold Flex® von BASF, Polyacrylate-21 (and) Acrylates/Dimethylaminoethyl Methacrylate Copolymer z.B. Syntran® PC5100 von Interpolymer, VP/Vinylcaprolactam/DMAPA Acrylates Copolymer z.B. Aquaflex® SF-40 von ISP, Polyquaternium-28 z.B. Gafquat® HS-100 von ISP, Polyquaternium-47 z.B. Merquat® 2001 von Nalco, Polyquaternium-55 z.B. Styleze® W-20 von ISP, Polyquaternium-69 z.B. Aquastyle® 300 von ISP oder VP/DMAPA Acrylates Copolymer (Copolymer vinylpyrrolidone and dimethylaminopropyl methacrylamide) z.B. Styleze® CC-1 0 von ISP.
Ebenfalls geeignet sind Polymere enthaltend Vinylimidazol bzw. das alkylierte (quaternisierte) Analogon. Hier sind z.B. VP/Methacrylamide/Vinyl Imidazole Copolymer z.B. Luviset® Clear von BASF, Polyquaternium-16 z.B. Luviquat® Typen von BASF, Polyquaternium-46 z.B. Luviquat® Hold von BASF oder Polyquaternium-68 z.B. Luviquat® Supreme von BASF als bevorzugte polymere Filmbildner zu nennen.
Ebenfalls geeignet sind Polymere enthaltend Diallyldimethylammonium Chlorid, wie z.B. Polyquaternium-22 z.B. Merquat® Typen von Nalco, Polyquaternium-39 z.B. Merquat® Typen von Nalco, Polyquaternium-4 z.B. Celquat® Typen von National Starch, Polyquaternium-6 z.B. Merquat® Typen von Nalco, Polyquaternium-7 z.B. Merquat® Typen von Nalco oder Salcare® Typen von Ciba oder Polyquaternium-87 z.B. Luviquat® Sensation von BASF.

Besonders bevorzugt umfasst mindestens ein filmbildendes Polymer als Monomer Dimethylaminoethylmethacrylat und mindestens ein filmbildendes Polymer zusätzlich mindestens ein Vinyllactam-Monomer.

Vorteilhaft ist weiterhin wenn mindestens ein filmbildendes Polymer ein Copolymer aus Vinylpyrrolidon, Vinylcaprolactam und Dimethylaminoethylmethacrylat ist.

Es ist erfindungsgemäß von Vorteil, wenn Mischungen von zwei oder mehreren der polymeren Filmbildner eingesetzt werden.

Als erfindungsgemäße polymere anionische Verdicker sind solche Substanzen zu verwenden, die ein Rheologie modifizierendes Polymer enthalten, welches als ein Monomer Acrylsäure enthält. Hierbei sind Polymere gemeint, die unter die Klasse der ASE- ("Alkali Swellable Emulsion"), bzw. HASE- ("Hydrophobically modified Alkali Swellable Emulsion") Verdicker fallen. Diese Verdicker können neben der Acrylsäure weitere, bevorzugt nichtionische, Monomere enthalten.

Bevorzugt ist mindestens ein anionisches verdickendes Polymer ein Copolymer aus Acrylsäure und Vinylpyrrolidon.

Besonders bevorzugte polymere anionische Verdicker sind hierbei Homopolymere der Acrylsäure mit der INCI-Bezeichnung Carbomer z.B. Carbopol® Typen von Lubrizol, Copolymere von Acrylsäure mit Alkylacrylaten mit der INCI-Bezeichnung Acrylates Copolymer z.B. Aculyn® 33 von Rohm&Haas, Copolymere von Acrylsäure mit Vinylpyrrolidon mit der INCI-Bezeichnung Acrylic Acid/VP Crosspolymer z.B. Ultrathix® P-100 von ISP, Copolymere von Acrylsäure mit C10-C30 Alkylacrylaten mit der INCI-Bezeichnung Acrylates/C10-30 Alkyl Acrylates Crosspolymer z.B. Carbopol® oder Pemulen® Typen Lubrizol oder Synthalen® Typen von 3V Sigma, Copolymere von Acrylsäure mit Ethylacrylat und assoziativen Alkylacrylaten mit der INCI-Bezeichnung Acrylates Copolymer z.B. Carbopol Aqua SF-1® von Lubrizol oder Copolymere von (Meth-)Acrylsäure mit Alkylacrylaten und ethoxylierten hydrophob modifizierten Alkylacrylaten mit den INCI-Bezeichnungen Acrylates/Steareth-20 Methacrylate Copolymer, Acrylates/Beheneth-25 Methacrylate Copolymer, Acrylates/Steareth-20 Methacrylate Crosspolymer z.B. Aculyn® 22, 28 oder 88 von Rohm&Haas oder der INCI Acrylates/Palmeth-25 Acrylates Copolymer z.B. Synthalen® von 3V Sigma.
Ebenfalls vorteilhaft können Verdicker auf Basis von Acrylamidomethyl Propansulfonsäure (AMPS), wie z.B. Ammonium Acryloyldimethyltaurate/VP Copolymer z.B. Aristoflex® AVC von Clariant oder Ammonium Acryloyldimethyltaurate/Beheneth-25 Methacrylate Copolymer z.B. Aristoflex® HMB von Clariant verwendet werden.

Es ist erfindungsgemäß von Vorteil, wenn Mischungen von zwei oder mehreren dieser polymeren anionischen Verdicker in der Zubereitung eingesetzt werden.

Es ist im Rahmen der Erfindung vorteilhaft, Wirkstoffe zur positiven Beeinflussung der Altershaut, die die Entstehung von Falten oder auch bestehende Falten vermindern, der erfindungsgemäßen Zubereitung zusätzlich zu zusetzen. Als besonders bevorzugte Wirkstoffe gelten daher Biochinone, insbesondere Ubichinon Q10, Isoflavon und Isoflavonoide, Genistein, Arctiin, Cardiolipin, Liponsäure, Anti Freezing Proteine, Hopfen- und Hopfen-Malz-Extrakte, und/oder die Restrukturierung des Bindegewebes fördernde Stoffe, Isoflavonoide sowie lsoflavonoid-haltige Pflanzenextrakte wie z.B. Soja- und Klee-Extrakte, die auch in den erfindungsgemäßen Zubereitungen und Hautauflagenmatrices sehr gut verwendet werden können. Auch zeigte sich, dass sich das erfindungsgemäße Kombinationsprodukt in besonderer Weise eignet, Wirkstoffe zur Unterstützung der Hautfunktionen bei trockener Haut, wie beispielsweise Vitamin C, Biotin, Kreatin, Kreatinin, Propionsäure, Glycerin, Grüntee-Extrakte, white tea - Extrakte oder - Lösungen, zu verwenden.

In ähnlicher Weise erweist sich die Einarbeitung von Wirkstoffen zur Linderung bzw. positiven Beeinflussung von irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süssholzes, Licochalcone A, Silymarin bzw.
Silyphos, Flavonoide, Dexpanthenol, Ethanol, Inhibitoren des Prostaglandinstoffwechsels, insbesondere der Cyclooxygenase, und des Leukotrienstoffwechsels, insbesondere der 5-Lipoxygenase, aber auch des 5-Lipoxygenase Inhibitor Proteins, FLAP.

Auch die Einarbeitung von Modulatoren der Pigmentierung ist erfindungsgemäß vorteilhaft. Hier sind Wirkstoffe zu nennen, die die Pigmentierung der Haut vermindern und so zu einer kosmetisch gewünschten Aufhellung der Haut führen und/oder das Auftreten von Altersflecken reduzieren und/oder bestehende Altersflecken aufhellen, wie Tyrosinsulfat, Vitamin C, Liponsäure und Liponamid, verschiedene Extrakte des Süssholzes, Kojisäure, Hydrochinon, Arbutin, Fruchtsäuren, insbesondere Alpha-Hydroxy-Säuren (AHAs), Bearberry (Uvae ursi), Ursolsäure, Ascorbinsäure, Grüntee-Extrakte, Aminoguanidin und/oder Pyridoxamin. In gleicher Weise erwiesen sich die erfindungsgemäßen Nahrungsergänzungsmittel, Zubereitungen oder Matrices als hervorragende Grundlage für Wirkstoffe, die eine verstärkte/schnellere Bräunung der Haut herbeiführen (Advanced Glycation Endproducts (AGE), Lipofuscine, Nukleinsäure-Oligonukleotide, Purine und Pyrimidine, NO-freisetzende Substanzen, sei es mit oder ohne Einfluss von UV-Licht.

Für den erfindungsgemäßen Einsatz unterstützend wirken vorteilhaft die Wirkstoffe, wie beispielsweise Folsäure, Phytoen, Harnstoff, D-Biotin, Coenzym Q10, Flavonglycoside wie z.B. α-Glucosylrutin, Carnitin, Polydocanol, natürliche und synthetische Isoflavonoide insbesondere Genistein, Flavonoide, Carotinoide, Kreatin, Kreatinin, Taurin, Ascorbinsäure sowie deren Derivate, Sauerstoff, Tocopherol sowie dessen Ester, Dihydroxyaceton, 8-Hexadecen-1,16-dicarbonsäure, langkettige Hyaluronsäuren (d.h. mit einem mittleren Motekuiargewicht von 1 Million bis 3 Million- Dalton) und kurzkettige Hyaluronsäuren (d.h. mit einem mittleren Molekulargewicht von 5000 bis 1 Million Dalton), Licochalcon A und deren Gemische.

Die zitierten Zusatzstoffe können jeweils zu einem Anteil von etwa 0,01 bis 30 Gew.%, bezogen auf das Gesamtgewicht der Zubereitung, in dieser enthalten sein.

Erfindungsgemäße Formulierungen, welche ein oder mehrere Antifaltenwirkstoffe wie Flavonglycoside insbesondere α-Glycosylrutin, Coenzym Q10, Retinol und dessen Ester, Vitamin E und dessen Derivate sowie andere dem Fachmann bekannte Antifaltenwirkstoffe enthalten, eignen sich zusätzlich insbesondere zum Schutz vor ästhetisch unattraktiven Hautveränderungen wie sie beispielsweise bei ermüdeter Haut oder der Hautalterung auftreten. Zu diesen Veränderungen gehören z.B. Trockenheit, Rauhigkeit und Ausbildung von Trockenheitsfältchen, Juckreiz, verminderte Rückfettung vor allem nach dem Waschen, sichtbare Gefäßerweiterungen wie Teleangiektasien oder Cuperosis, Schlaffheit und Ausbildung von Falten und Fältchen, lokale Hyper-, Hypo- und Fehlpigmentierungen wie Altersflecken, vergrößerte Anfälligkeit gegenüber mechanischem Stress wie Rissigkeit und andere dem Fachmann bekannte Veränderungen. Weiterhin vorteilhaft eignen sich die erfindungsgemäßen Emulsionen gegen das Erscheinungsbild der trockenen bzw. rauhen Haut.

Weiterhin vorteilhaft ist der Einsatz von ein oder mehrere Antioxidantien in erfindungsgemäßen Rezepturen. Als geeignete Antioxidantien können ein oder mehrere Substanzen gewählt werden aus der Gruppe enthaltend Aminosäuren und deren Derivate wie z.B. Glycin, Histidin, Tyrosin und Tryptophan, Imidazole und deren Derivate wie z.B. Urocaninsäure, Peptide und deren Derivate wie z.B. D,L-Carnosin, D-Carnosin, L-Carnosin und Anserin, Carotinoide, Carotine und deren Derivate wie z.B. α-Carotin, β-Carotin und Lycopin, Liponsäure und deren Derivate wie z.B. Dihydroliponsäure, Aurothioglucose, Propylthiouracil und andere Thiole sowie deren Salze wie z.B. Thioredoxin, Glutathion, Cystein, Cystin, Cystamin sowie deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl - und Glycerylester, Dilaurylthiodipropionat, Distearylthiodipropionat, Thiodipropionsäure und deren Derivate wie Ester, Ether, Peptide, Lipide, Nukleotide, Nukleoside und Salze, Sulfoximinverbindungen wie z.B. Buthioninsulfoximine, Homocysteinsulfoximin, Buthioninsulfone, Penta-, Hexa- und Heptathioninsulfoximin in sehr geringen verträglichen Dosierungen (z.B. pmol bis µmol/kg), ferner (Metall)-Chelatoren wie z.B. α-Hydroxyfettsäuren, Palmitinsäure, Phytinsäure und Lactoferrin, α-Hydroxysäuren wie z.B. Zitronensäure, Milchsäure und Apfelsäure, Huminsäure, Gallensäürer, Gallenextrakte, Bilirubin, Biliverdin, EDTA, EGTA und deren Derivate, ungesättigte Fettsäuren und deren Derivate wie z.B. γ-Linolensäure, Linolsäure und Ölsäure, Folsäure und deren Derivate, Ubichinon und Ubichinol und deren Derivate, Vitamin C und dessen Derivate wie z.B. Ascorbylpalmitat, Mg-Ascorbylphosphat und Ascorbylacetat, Tocopherole und deren Derivate wie z.B. Vitamin E-Acetat, Vitamin A und dessen Derivate wie z.B. Vitamin A-Palmitat, Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate wie z.B. ZnO und ZnSO₄, Selen und dessen Derivate wie z.B. Selenmethionin, Stilbene und deren Derivate wie z.B. Stilbenoxid und trans-Stilbenoxid, sowie die erfindungsgemäß geeigneten Derivate der genannten Wirkstoffe wie Salze, Ester, Ether, Zucker, Nukleotide, Nukleoside, Peptide oder Lipide.

Die Menge der Antioxidantien (eine oder mehrere Verbindungen) in den erfindungsgemäßen Formulierungen beträgt vorzugsweise 0,001 bis 30 Gew.%, besonders bevorzugt 0,05 bis 20 Gew.%, insbesondere 0,1 bis 10 Gew.%, bezogen auf das Gesamtgewicht der Zubereitung.

Sofern Vitamin E oder dessen Derivate das oder die Antioxidantien darstellen, ist es vorteilhaft, deren jeweiligen Anteil im Bereich von 0,001 bis 10 Gew.%, bezogen auf das Gesamtgewicht der Zubereitung, zu wählen. Sofern Vitamin A, Vitamin-A-Derivate, Carotine oder deren Derivate das oder die Antioxidantien darstellen, ist es vorteilhaft, deren jeweiligen Anteil im Bereich von 0,001 bis 10 Gew.-%, bezogen auf das Gesamtgewicht der Zubereitung, zu wählen.
Es ist insbesondere vorteilhaft, wenn die kosmetischen Zubereitungen als Emulsionen gemäß der vorliegenden Erfindung kosmetische Wirkstoffe enthalten, wobei bevorzugte Wirkstoffe Antioxidantien sind, welche die Haut vor oxidativer Beanspruchung schützen können.

Zudem können die erfindungsgemäßen Formulierungen weitere, in kosmetischen Formulierungen bekannte Inhaltsstoffe enthalten, wie z.B. Acetyl Trifluoromethylphenyl Valylglycine, Acrylamide / Ammonium Acrylate Copolymer, Acrylates/C12-22 Alkylmethacrylate Copolymer, Aluminum / Magnesium Hydroxide Stearate, Ammonium Lactate, Ammonium Polyacrylate, Ammonium Polyacryloyldimethyl Taurate, Arginine PCA, Beerwax, Benzethoniumchlorid, Capryloyl Salicylic Acid, β-Carotin, Cinnamic Acid, Coco Glucoside, Copper Gluconate, Dehydroxanthan Gum, Diphenyl Dimethicone, Disodium Adenosine Triphosphate, Disodium Succinate, Disteardimonium Hectorite, Dodecene, Eperua Falcata, Hydrogenated Palm Glycerides Citrate, Hydrogenated Palm Kernel Glycerides, Hydrolyzed Wheat Protein, PG-Propyl Methylsilanediol, Kasei, Kinetin, Hydroxyethyl Acrylate / Sodium Acryloyldimethyltaurate Copolymer, Isodeceth-6, Linseed Acid, Lutein, Lyopene, Magnesium Aspartate, Melibiose, Oxothiazolidinecarboxylic Acid, Palmitoyl Pentapeptide 4, PEG-8 Laurate, PG-10 Stearat, Phenethyl Alcohol, Phenylpropanol, Polyacrylate-13, Polyacrylate-3, Quinic Acid, Sarcosine, Saxifraga Sarmentosa Extract, Scutellaria Baicalensis Extract, Shikimic Acid, Sodium Metabisulfite, Soy Isoflavone, Tocopheryl Glucoside, Trideceth-6, Zeaxanthin, Zinc Gluconate, Triacetin, 1,2 Hexandiol, Hydroxyethylpiperazine Ethane Sulfonic Acid, Nicotinamide, Penethyl Alcohol, Penthylene Glycol, Carnaubau Wax, Chlorhexidine Digluconate, Oleyl Erucate, Acetyltrifluoromethylphenylvalylglycin, Acrylamidammoniumacrylatcopolymer, Aluminummagnesiumhydroxidstearat, Ammoniumlactat, Ammoniumpolyacrylat, Ammoniumpolyacryloyldimethyltaurat, Arginin PCA, Capryloylsalicylsäureester, Zimtsäure, Cocoglucosid, Kupfergluconat, Diphenyldimethicon, Dinatriumadenosintriphosphat, Dnatriumsuccinat, Disteardimoniumhectorit, Dodecen, Eperua Falcata , hydriertes Palmenglycerid, hydriertes Palmenglyceridcitrat, Hydrierte Palmenkernglyceride, Hydrolisiertes Weizenprotein PG-Propylmethylsilandiol, Hydroxyethylacrylat / Natriumacryloyldimethyltauracopolymer, Isodeceth-6, Linseed Acid, Magnesiumaspartat, Melibiose, Oxothiazolidinecarboxylsäure, Palmitoylpentapeptide 4, PEG-8 Laurat, Phenethylalkohol, Phenylpropanol, Polyacrylate-13, Polyacrylate-3 , Sarcosin, Saxifraga Sarmentosa Extrakt, Scutellaria Baicalensis Extrakt , Natriummetabisulfit, Sojaisoflavon, Tocopherylglucosid, Trideceth-6, Zinkgluconat , Triacetin, 1,2 Hexandiol, Hydroxyethylpiperazine Ethane Sulfonic Acid, Nicotinamide, Penethyl Alcohol, Penthylene Glycol, Carnaubau Wax, Chlorhexidine Digluconate und/oder Oleyl Erucate.

Zudem ist die Verwendung von Stoffen zur positiven Beeinflussung des Geruchs der Formulierung sinnvoll. Hierzu zählen z.B. Dipropylene glycol, Methyl dihydrojasmonate, Phenethyl alcohol, Linalool, Linalyl acetate, 2,6-Dimethyl-7-octen-2-ol, alpha-Hexylcinnamaldehyde, 2-Acetonapthone-1,2,3,4,5,6,7,8-octahydro-2,3,8,8-tetramethyl p-t-Butylalpha-methyldihydrocinnamic aldehyde, Benzyl acetate, 1,3,4,6,7,8-Hexahydro-4,6,6,7,8,8-hexamethylcyclopenta-gamma-2-benzopyran, Methyl cedryl ketone, Ethylene brassylate, 4-(4-Hydroxy-4-methylpentyl)-3-cyclohexene-1-carboxyaldehyde, Benzyl salicylate, Hexyl salicylate Orange oil, alpha-Isomethylionone, Diethyl phthalate, 4-t-Butylcyclohexyl acetate, Patchouli oil 3,7-Dimethyl-2,6-octadien-1-ol, Tetrahydrolinalool, Hydroxycitronellal, Isopropyl myristate, 3,7-Dimethyl-6-octen-1-ol, Orange terpenes, Heliotropin, Terpinyl acetate, omega-Pentadecalactone, Methyl-alpha-ionone, Lavandin oil, Lemon oil, Bergamot oil, 7-Acetyl-1,1,3,4,4,6-hexamethyltetralin, Coumarin, Ethyllinalool, Amyl salicylate, 2-tert-Pentyl-cyclohexyl acetate, 3-Methyl-5-phenyl-1-pentanol, Cedrol, Benzyl benzoate, Vanillin, alpha-Amylcinnamaldehyde, Dimethyl phthalate, d-Limonene, 2-Isobutyl-4-hydroxy-4-methyttetrahydropyran, Triethyl citrate, Terpineol, Lavender oil, Diethylene glycol monoethyl ether, 2-Phenoxyethyl isobutyrate, Anisyl alcohol, 3-Perrtyltetrahydro(2H)pyranyl acetate, Methyl ester of rosin, partially hydrogenated, lsobornyl acetate, Rosemary oil, Petitgrain oil, 1,4-Dioxacyclohexadecane-5,16-dione, Isoamyl salicylate, gamma-Undecalactone, alpha-lonone, Oxacyclohexadecen-2-one, 7-Octen-2-ol, 2-methyl-6-methylene, dihydro derive. 1,2-Propylene glycol, 3-(5,5,6-Trimethylbicyclo(2.2.1)hept-2-yl)cyclohexan-1-ol, Geranium oil, Musk ketone, Cedrenyl acetate, Isobornylcyclohexanol, lonone, Benzyl alcohol, gamma-Nonalactone, I-Menthol, Cyclohexyl salicylate, Dihydromyrcenyl acetate, Citral, Orange terpenes (natural), Cedarwood oil, alpha-Pinene, Majantol, Phenoxyethanol, Ethyl acetate, Cedrol methyl ether, 1,5,9-Trimethyl-13-oxabicyclo(10.1.0) trideca-4,8-diene, Peppermint oil, Eugenol, Ethyl maltol, Benzaldehyde, Cinnamic alcohol, 3,7-Dimethyl-1-octanol, alpha-Methyl-3,4-methylene dioxyhydrocinnamic aldehyde, beta-Pinene, d-Camphor, Methyl abietate, Cedryl acetate, Ylang ylang oil, Sandalwood oil, Mineral oil, Dimethyl benzyl carbinyl butyrate, Ethyl butyrate, Geranyl acetate, Hexylene glycol, Myrcene, alpha-Methyionantheme, beta-lonone, 3-(4-t-Butylphenyl)propanal, 3,7-Dimethyloctan-3-yl acetate, Acetic acid, (1-oxopropoxy)-,1-(3,3-dimethylcyclohexyl), Eucalyptol, 4-Carvomenthenol, Stearic acid, Menthanyl acetate, Eucalyptus oil, Dihydroterpinyl acetate, o-t-Butylcyclohexyl acetate, Isoeugenol, alpha-Terpineol, Cyclamen aldehyde, Hydroxycitronellol, Myrcenyl acetate, Nopyl acetate, 3,7-Dimethyl-1,3,6-octatriene, Rhodinol, Dimethyl benzyl carbinyl acetate, Tricyclodecenyl propionate, 2-Methyl-5-phenylpentan-1-ol, Sclareoate, 3-Isocamphyl cyclohexanol, trans-Anethole, Hexahydro-4,7-methanoinden-5(6)-yl acetate, 4-(p-Hydroxyphenyl)-2-butanone, Nerolidol, alpha-Butylcinnamaldehyde, Bornyl acetate, Etyhl methylphenylglycidate, trans-beta-lonone, Camphene, Juniper berry oil, Mandarin oil, Nutmeg oil, Spearmint oil, Grapefruit oil, Labdanum oil, Galbanum oil, Menthone, Trichloromethyl phenyl carbinyl acetate, alpha-Methylbenzyl acetate, Ethyl-2methyl-1,3-dioxolane-2-acetate, 2,6-Nonadienal, Abietyl acetate, Anisic acid, Diphenyl ether, Triacetin, 2-Methyl-4-phenyl-2-butanol, Phenylethyl acetate, 1-Phenyl-3-methyl-3-pentanol, Anisyl acetate, Cinnamic aldehyde, p-Methylanisole, 5-Phenylpentanol, Diethyl malonate, Citronellal, Nerol, Undecanal, 2-methyl-, Hexyl alcohol, Glyceryl caprylate, Methyl 2-nonenoate, Octyl acetate, Decanal, Lauryl alcohol, Lauric aldehyde, Ethyl vanillin, 3-Phenyl-1-propanol, Octanal, Butylated hydroxytoluene, 4-Acetyl-6-t-butyl-1,1-dimetylindane, delta-3-Caren, Benzyl laurate, Neryl acetate, Ethyl acetoacetate, Hexyl acetate, Menthol liquid, Citronellyl acetate, Tetrahydromyrcenol, Diacetin, Menthyl acetate, 3(4),8(9)-Dihydroxymethyl tricyclo(5.2.1.0(2,6)decane, 2,4-Dimethyl-3-cyclohexen-1-carboxaaldehyde, Cedrenol, Phenylacetaldehyde glyceryl acetal, Sabinene, 3,7,11-Trimethyl-1,2,10,-dodecatrien-3-ol (cis & trans), Octyldodecanol, Formaldehyde cyclododecyl ethyl acetal, Myristicin, 3,7-Dimethyl-2(3),6-nonadienenitrile, Ethyllinylyl acetate, 2-Methylbotyl acetate, cis-3-Hexenyl salicylate, 2-Methyl-4-(2,6,6-trimethyl-2(1)-cyclohexen-1-yl) butanal, Maltol isobutyrate, 2-Methyl-3(4-(2-methylpropyl)phenyl)propanal, 12-Oxahexadecanolide, 1,1-Dimethoxy-2,25-trimethyl-4-hexene, 1,6,7,8-Tetrahydro-1,4,6,6,8,8-hexamthyl-as-indacen-3<2H>-one, Bergamot oil, bergaptene free, Treemoss abs., Citrus oil distilled, Lemon terpenes, gamma-Decalactone, 2-Methyl-4-phenyl-2-pentanone, Allyl phenoxyacetate, Methyl-delta-ionone, Citronella oil, Clove bud oil, Thyme oil, Lime oil, Bois de rose oil, Cognac oil, Neroli bigarade oil, Spike lavender oil, Vetiver oil, Fir needle oil, Methylpentenolone, Lemon oil terpenes, Isobutyl salicylate, beta-Caryophyllene, Pulegone, Thymol und/oder gamma-Terpinene.

Es ist zudem von Vorteil, wenn die Zubereitung einen oder mehrere UV-Filter enthält, gewählt aus der Gruppe der Verbindungen Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon; 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxy-siloxan / Dimethylsiloxan - Copolymer; Dioctylbutylamidotriazon (INCI: Diethylhexyl-Butamidotriazone; 2,4-bis-[5-1(dimethylpropyl)benzoxazol-2-yl-(4-phenyl)-imino]-6-(2-ethylhexyl)-imino-1,3,5-triazin) (mit der CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin) (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,2'-Methylen-bis-(6-(2H-benzotriazol-2-yl)-4-(1,1,3,3-tetramethylbutyl)-phenol); Polysilicone-15; 2,4-Bis-(4'-Di-neopentylaminobenzalmalonat)-6-(4"-butylaminobenzoat)-s-triazin; Merocyanine; 2,4,6-Tris-(biphenyl)-,3,5-triazin; Phenylbenzimidazolsulfonsäure und/oder deren Salze; 2-(4'-Diethylamino-2'-hydoxybenzoyl)-benzoesäurehexylester; Zinkoxid; Titandioxid.

Besonders vorteilhaft sind zusätzlich UV-Filter, aktive Wirkstoffe zur Minderung von Falten, Co-Lösungsmittel, Feuchthaltemittel, Pflanzenextrakte, Vitamine, Antioxidantien, Emulgatoren, Tenside, Öle, Fette, Wachse, Silikone, Konservierungsmittel und/oder Parfümstoffe in der Zubereitung enthalten.

Diese Stoffe lassen sich in der für den Effekt erforderlichen Konzentration zu den Formulierungen zufügen, ohne dabei einen negativen Einfluss auf den Straffungseffekt, die Produktsensorik oder Stabilität auszuüben.

Die erfindungsgemäße Zubereitung ist bevorzugt als Kosmetikum formuliert.

Auch als dermatologische Zubereitung mit dann entsprechenden therapeutisch wirksamen Inhaltsstoffen ist erfindungsgemäß, ebenso wie deren Verwendung zur Herstellung pharmazeutischer Produkte.

De Zubereitung kann sowohl als Gel, Hydrodispersion als auch emulsionsbasierend formuliert werden.
Die erfindungsgemäße Zubereitung ist vorteilhaft **dadurch gekennzeichnet, das** sie in Form einer wässrigen oder wässrig-alkoholischen Lösung, einer Emulsion (W/O, O/W, W/Si, Si/W oder multiple Emulsion, Makro-, Mikro- oder Nanoemulsion), einer Dispersion, einer Pickering-Emulsion, eines Gels oder eines Hydrodispersionsgels vorliegt.
Die Zubereitung kann erfindungsgemäß auch in Form einer dünnflüssigen, sprühfähigen wässrigen oder wässrig-alkoholischen Lösung, in Form eines Gels, in einer Wachsmatrix, einer Stiftform, als Salbe, Creme oder Lotion (ggf. sprühbar) vorliegen.
Die Zubereitung kann erfindungsgemäß vorteilhaft auch als Aerosol, Spray oder als Tränkungsmedium für ein Tuch eingesetzt werden. Daher sind auch Tücher getränkt mit der erfindungsgemäßen Zubereitung, erfindungsgemäß.

Bevorzugt sind hierbei wässrige Gele und Hydrodispersionsgele, sowie O/W oder Si/W Emulsionen.

Die erfindungsgemäßen Zusammensetzungen können ferner gegebenenfalls in der Kosmetik übliche Zusatzstoffe, beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten.
Moisturizer können vorteilhaft auch als Antifaltenwtrkstoffe zum Schutz vor Hautveränderungen, wie sie z. B. bei der Hautalterung auftreten, verwendet werden

Wesentlicher Vorteil der erfindungsgemäßen Zubereitung ist, dass sie einen sofort spürbaren Straffungseffekt erzeugt.

Die hautstraffende Wirkung wurde in einem Paneltest mit mehr als 10 trainierten Panelisten bewertet. Dazu wurden 0,20 g einer erfindungsgemäßen Formulierung (siehe Beispiele) gleichmäßig auf dem Handrücken verteilt. Nach bzw. während der Trocknungsphase von maximal 3 Minuten wurde der straffende Effekt von den Panelisten bewertet. Durch Bewegung der Hand und der damit verbundenen Bewegung der Haut auf dem Handrücken können dabei auch geringe Unterschiede im Straffungsgefühl ermittelt werden.
Die Panelisten bewerteten die Straffung auf einer Skala von 0 (keine Straffung) bis 6 (maximale Straffung). Zum Vergleich wurde den Panelisten Hansaplast Sprühpflaster appliziert, eine bekanntermaßen klebende Formulierung, mit der die maximale Straffung von 6 erreicht wird. Erfindungsgemäße Formulierungen müssen in diesem Test einen Mittelwert der 10 Panelisten von mindestens 2 erreichen.

**Ergebnis:Sofort-Straffungseffekt**

| | | | | | | |
|---|---|---|---|---|---|---|
| Beispiel | 1 | 2 | 3 | 4 | 5 | 6 |
| Straffungseffekt | 3,0 | 3;2 | 5,2 | 3,5 | 4,9 | 4,1 |

Zubereitungen ohne die jeweilige erfindungsgemäße Kombination, also entweder ohne polymeren Filmbildner mit ein oder mehrere kationische und/oder kationogene Monomer oder ohne polymeren anionischen Verdicker, zeigten keinen Straffungseffekt.

Die Verwendung der Kombination von polymeren Filmbildner, die ein oder mehrere kationische und/oder kationogene Monomere enthalten, und polymere anionische Verdicker, bevorzugt solche die als ein Monomer Acrylsäure enthalten, in kosmetischen oder dermatologischen Zubereitungen zur Hautstraffung ist somit predestiniert.
Als Sofort-Straffungseffekt wird eine Hautstraffung innerhalb von 3 Minuten nach Hautauftrag bezeichnet, wie im Paneltest gezeigt.
Die Verwendung umfasst bevorzugt polymere Filmbildner gewählt aus Vinylcaprolactam/VP/Dimethylaminoethylmethacrylate Copolymer, Polyquaternium-11, Polyquaternium-89, Polyacrylate-21 (and) Acrylates/Dimethylaminoethyl Methacrylate Copolymer, VP/Vinylcaprolactam/DMAPA Acrylates Copolymer, Polyquaternium-28, Polyquaternium-47, Polyquaternium-55, Polyquaternium-69, VP/DMAPA Acrylates Copolymer, VP/Methacrylamide/Vinyl Imidazole Copolymer, Polyquaternium-16, Polyquaternium-46, Polyquaternium-68, Polyquaternium-22, Polyquaternium-39, Polyquaternium-4, Polyquaternium-6, Polyquaternium-7 und/oder Polyquaternium-87
sowie als polymere anionische Verdicker gewählt aus
Carbomeren, Copolymere von Acrylsäure mit Alkylacrylaten mit der INCI-Bezeichnung Acrylates Copolymer, Copolymere von Acrylsäure mit Vinylpyrrolidon mit der INCI-Bezeichnung Acrylic AcidNP Crosspolymer, Copolymere von Acrylsäure mit C10-C30 Alkylacrylaten mit der INCI-Bezeichnung Acrylates/C10-30 Alkyl Acrylates Crosspolymer, Copolymere von Acrylsäure mit Ethylacrylat und assoziativen Alkylacrylaten mit der INCI-Bezeichnung Acrylates Copolymer (Carbopol Aqua SF-1® von Lubrizol) oder Copolymere von (Meth-)Acrylsäure mit Alkylacrylaten und ethoxylierten hydrophob modifizierten Alkylacrylaten mit den INCI-Bezeichnungen Acrylates/Steareth-20 Methacrylate Copolymer, Acrylates/Beheneth-25 Methacrylate Copolymer, Acrylates/Steareth-20 Methacrylate Crosspolymer oder der INCI Acrylates/Palmeth-25 Acrylates Copolymer, Acrylamidomethyl Propansulfonsäure, Ammonium Acryloyldimethyltaurate/VP Copolymer und/oder Ammonium Acryloyldimethyltaurate/Beheneth-25 Methacrylate Copolymer.

Es ist erfindungsgemäß von Vorteil, wenn Mischungen von jeweils zwei oder mehreren der polymeren Filmbildner bzw. anionischen Verdickern verwendet werden.
Die erfindungsgemäße Kombination ist in dermatologischen Zubereitungen zu deren Herstellung zu verwenden.

Es ist erfindungsgemäß vorteilhaft die Zubereitungen umfassend die Kombinationen aus polymeren Filmbildner, die ein oder mehrere kationische und/oder kationogene Monomere enthalten, und polymere anionische Verdicker zur Anwendung auf der menschlichen Haut zur Erzielung eines sofort spürbaren Hautstraffungseffekt zu verwenden. "Sofort spürbar" ist im Sinne der Erfindung derart zu verstehen, dass innerhalb von 3 Minuten nach Auftragen des Produktes vom geschulten Panel ein deutlicher Straffungseffekt wahrgenommen wird.

Als Verwendung der Zubereitung zur Straffung der Haut ist auch die Verwendung zur Festigung der Haut umfasst und erfindungsgemäß.
Als bevorzugtes Anwendungsgebiet der hautstraffenden Wirkung ist die Verwendung der Zubereitung zur kosmetischen und/oder dermatologischen Behandlung und/oder Prophylaxe der Cellulite geeignet.

Die nachfolgenden Beispiele stellen bevorzugte erfindungsgemäße Zubereitungen dar. Die Zahlenangaben sind Gewichtsanteile bezogen auf de Gesamtmasse der Zubereitung.
Bei allen Beispielrezepturen wurde vom Experten-Panel ein Straffungseffekt von mindestens 2 auf der Skala von 0 bis 6 festgestellt.

Beispiele für Formulierungen mit sofort spürbarem Straffungs-Effekt.

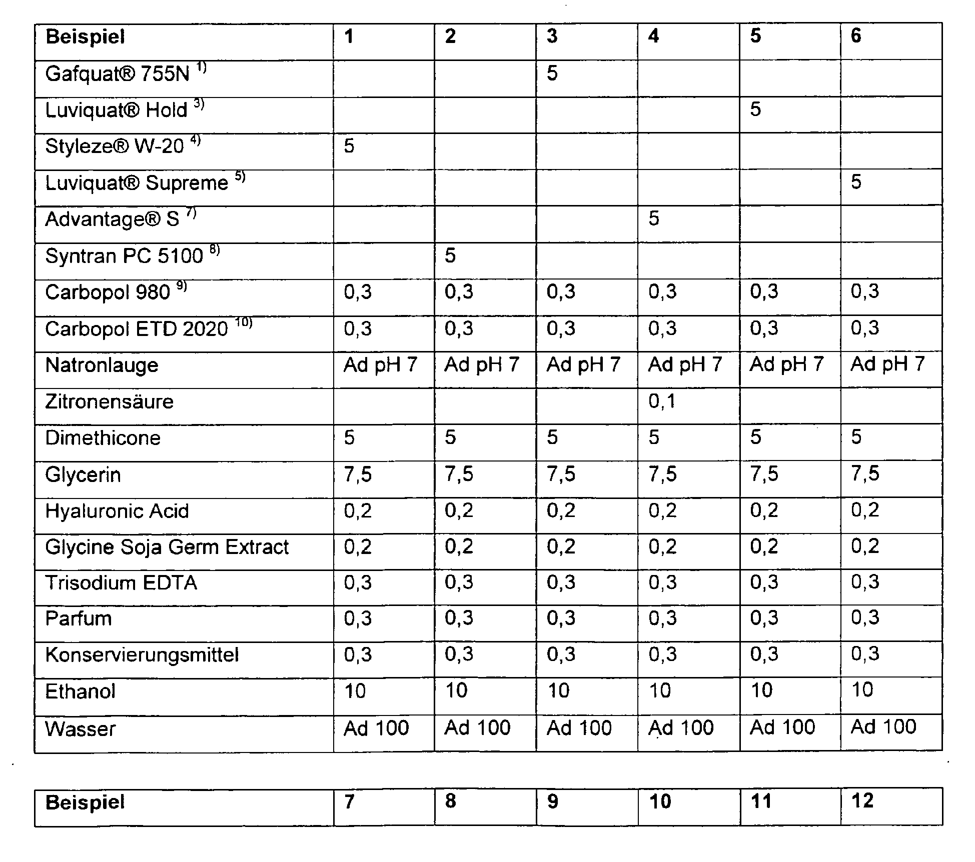

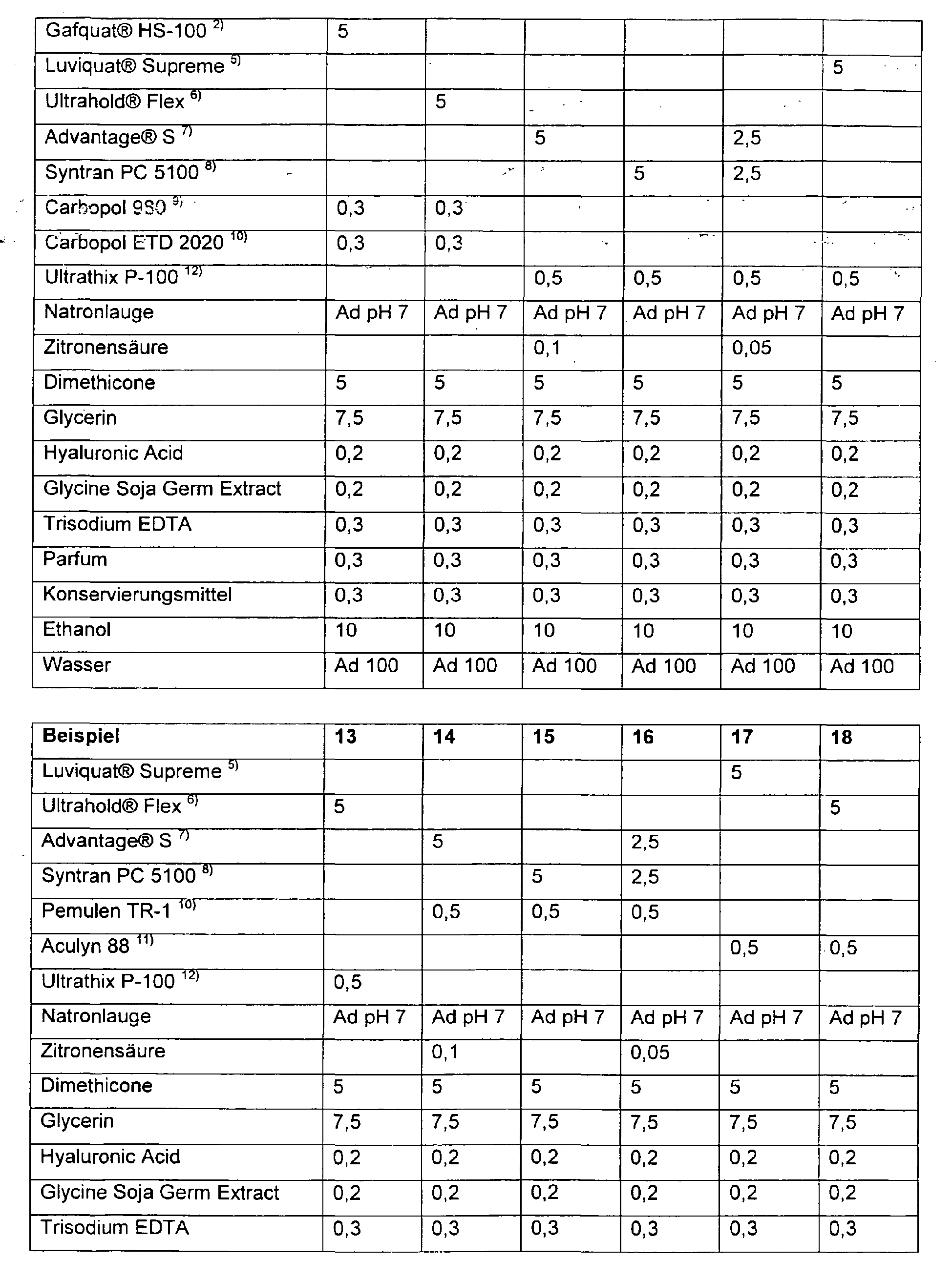

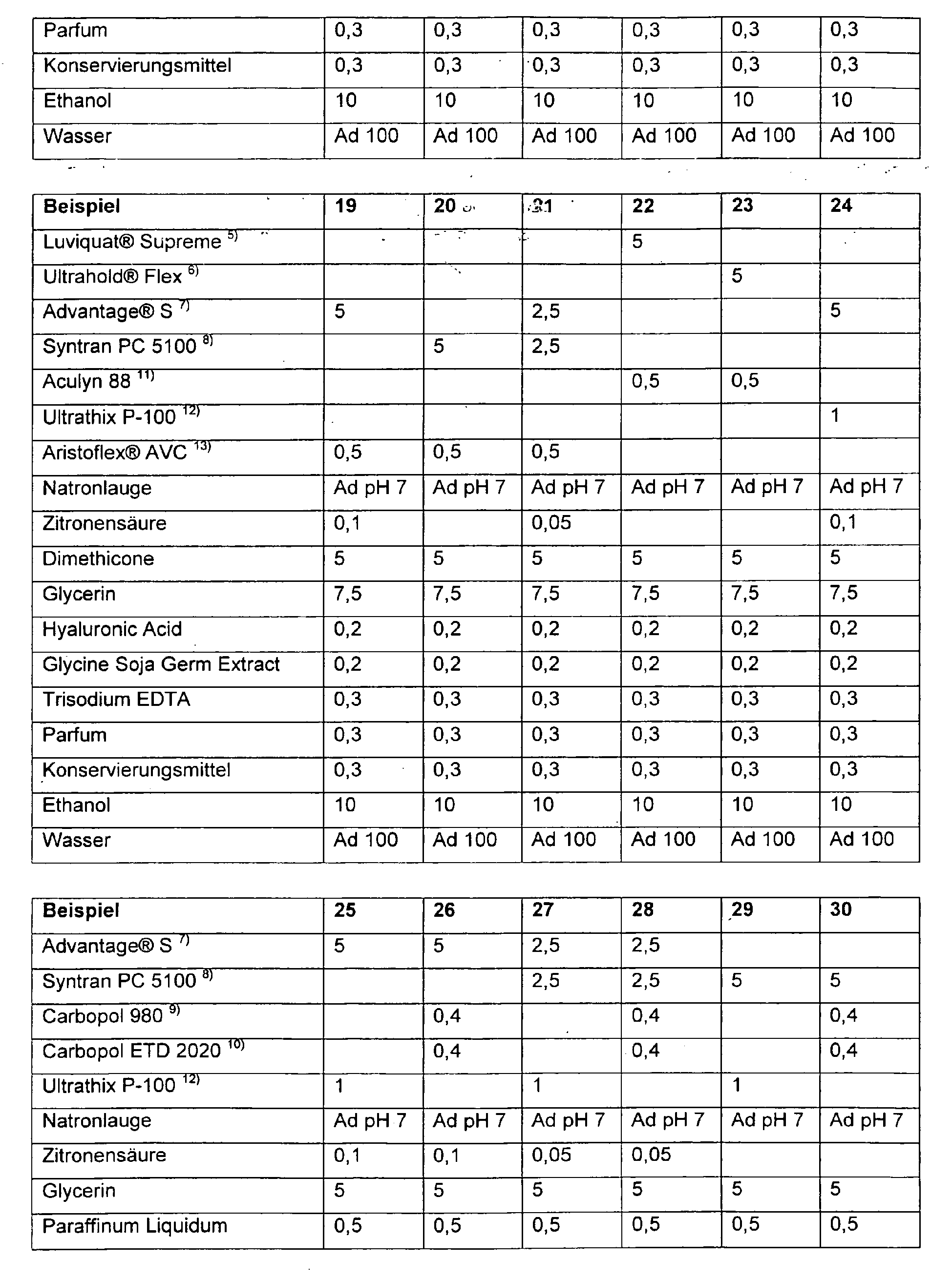

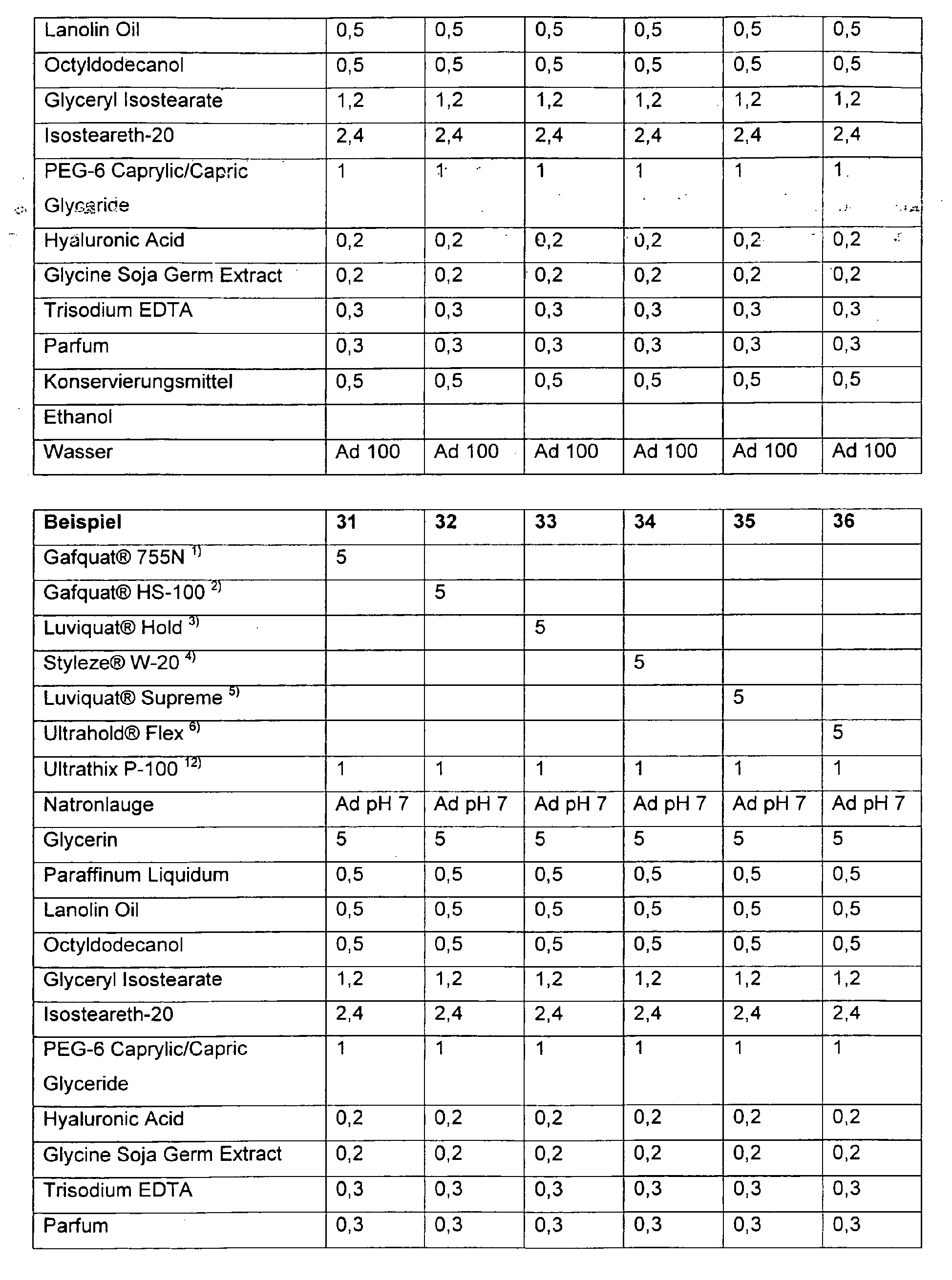

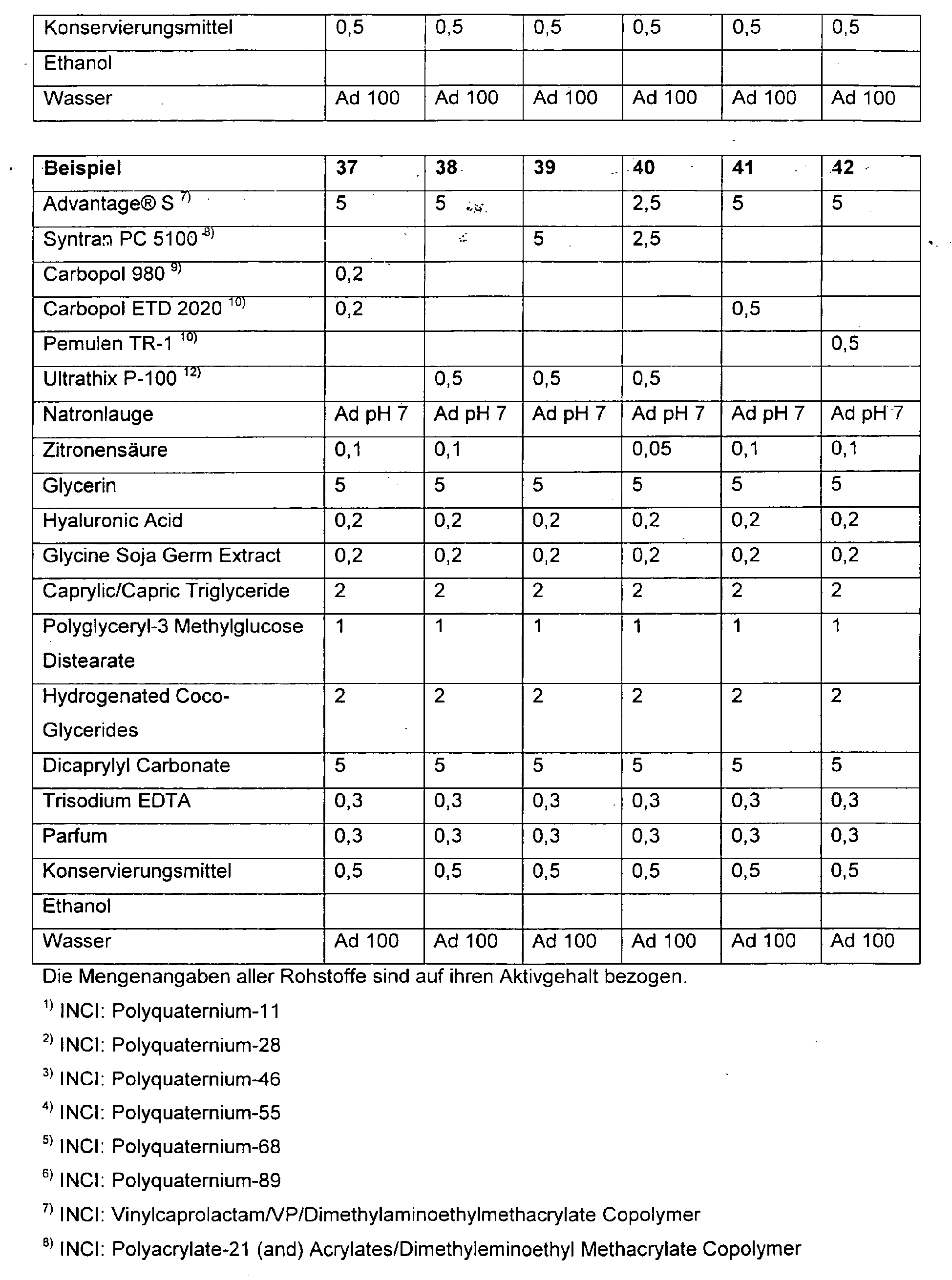

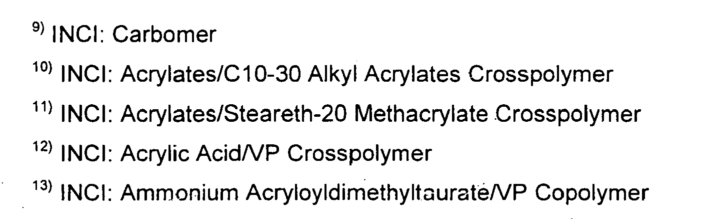

Alle Beispielzubereitungen zeigen nach dem Aufbringen auf die Haut einen sofort spürbaren Straffungseffekt.

## Patentansprüche

1. Verwendung der Kombination von mindestens einem polymeren Filmbildner, der ein oder mehrere kationische und/oder kationogene Monomere enthält, und mindestens einem polymeren anionischen Verdicker in kosmetischen Zubereitungen zur Hautstraffung.

2. Verwendung der Kombination nach Anspruch 1 zur Herstellung einer dermatologischen Zubereitung zur Hautstraffung.

3. Verwendung nach einem der vorhergehenden Ansprüche, **dadurch gekennzeichnet, dass** der oder die polymeren Filmbildner gewählt werden aus Polymeren, die ein oder mehrere kationische und/oder kationogene Monomere enthalten, die eine kationische Ladung im Bereich von pH 2 bis pH 12 unabhängig vom pH-Wert tragen und/oder die eine oder mehrere protonierbare Gruppe haben, die bei pH-Werten zwischen 2 und 12 protoniert vorliegen.

4. Verwendung nach einem der vorhergehenden Ansprüche, **dadurch gekennzeichnet, dass** der oder die polymeren Filmbildner gewählt werden aus Vinylcaprolactam/VP/Dimethylaminoethylmethacrylate Copolymer, Polyquaternium-11, Polyquaternium-89, Polyacrylate-21/Acrylates/Dimethylaminoethyl Methacrylate Copolymer, VP/Vinylcaprolactam/DMAPA Acrylates Copolymer, Polyquaternium-28, Polyquaternium-47, Polyquaternium-55, Polyquaternium-69, VP/DMAPA Acrylates Copolymer, VP/Methacrylamide/Vinyl Imidazole Copolymer, Polyquaternium-16, Polyquaternium-46, Polyquaternium-68, Polyquaternium-22, Polyquaternium-39, Polyquaternium-4, Polyquaternium-6, Polyquaternium-7 und/oder Polyquaternium-87.

5. Verwendung nach einem der vorhergehenden Ansprüche, **dadurch gekennzeichnet, dass** der oder die polymeren anionischen Verdicker gewählt werden aus Polymeren umfassend als ein Monomer Acrylsäure.

6. Verwendung nach einem der vorhergehenden Ansprüche, **dadurch gekennzeichnet, dass** der oder die polymeren anionische Verdicker gewählt werden aus Carbomeren, Copolymere von Acrylsäure mit Alkylacrylaten mit der INCI-Bezeichnung Acrylates Copolymer, Copolymere von Acrylsäure mit Vinylpyrrolidon mit der INCI-Bezeichnung Acrylic Acid/VP Crosspolymer, Copolymere von Acrylsäure mit C10-C30 Alkylacrylaten mit der INCI-Bezeichnung Acrylates/C10-30 Alkyl Acrylates Crosspolymer, Copolymere von Acrylsäure mit Ethylacrylat und assoziativen Alkylacrylaten mit der INCI-Bezeichnung Acrylates Copolymer oder Copolymere von (Meth-)Acrylsäure mit Alkylacrylaten und ethoxylierten hydrophob modifizierten Alkylacrylaten mit den INCI-Bezeichnungen Acrylates/Steareth-20 Methacrylate Copolymer, Acrylates/Beheneth-25 Methacrylate Copolymer, Acrylates/Steareth-20 Methacrylate Crosspolymer oder der INCI Acrylates/Palmeth-25 Acrylates Copolymer, Acrylamidomethyl Propansulfonsäure, Ammonium Acryloyldimethyltaurate/VP Copolymer und/oder Ammonium Acryloyldimethyltaurate/Beheneth-25 Methacrylate Copolymer.

7. Verwendung nach einem der vorhergehenden Ansprüche, **dadurch gekennzeichnet, dass** das filmbildende Polymer zu einem Anteil von 0,1 bis 60 Gew.%, bevorzugt zu einem Anteil von 0,5 bis 30 Gew.% und besonders bevorzugt zu einem Anteil von 1 bis 10 Gew.% bezogen auf die Gesamtmasse der Zubereitung, eingesetzt wird.

8. Verwendung nach einem der vorhergehenden Ansprüche, **dadurch gekennzeichnet, dass** der polymere anionische Verdicker zu einem Anteil von 0,02 bis 60 Gew.%, bevorzugt zu einem Anteil von 0,1 bis 30 Gew.% und besonders bevorzugt zu einem Anteil von 0,2 bis 2,5 Gew.% bezogen auf die Gesamtmasse der Zubereitung, eingesetzt wird.

9. Verwendung nach einem der vorhergehenden Ansprüche, **dadurch gekennzeichnet, dass** das filmbildende Polymer zum verdickenden Polymer im Verhältnis von 5:1 bis 1:5 eingesetzt wird.

10. Verwendung nach einem der vorhergehenden Ansprüche, **dadurch gekennzeichnet, dass** das kationische und/oder kationogene filmbildende Polymer wasserlöslich ist.

11. Verwendung nach einem der vorhergehenden Ansprüche, **dadurch gekennzeichnet, dass** die Zubereitungen als wässrige Gelen, Hydrodispersionen oder O/W Emulsionen vorliegen.

12. Verwendung nach einem der vorhergehenden Ansprüche, **dadurch gekennzeichnet, dass** mindestens ein filmbildendes Polymer als Monomer Dimethylaminoethylmethacrylat enthält.

13. Verwendung nach einem der vorhergehenden Ansprüche, **dadurch gekennzeichnet, dass** mindestens ein filmbildendes Polymer zusätzlich mindestens ein Vinyllactam-Monomer enthält.

14. Verwendung nach einem der vorhergehenden Ansprüche, **dadurch gekennzeichnet, dass** mindestens ein filmbildendes Polymer ein Copolymer aus Vinylpyrrolidon, Vinylcaprolactam und Dimethylaminoethylmethacrylat ist.

15. Verwendung nach einem der vorhergehenden Ansprüche, **dadurch gekennzeichnet, dass** mindestens ein anionisches verdickendes Polymer ein Copolymer aus Acrylsäure und Vinylpyrrolidon ist.

16. Verwendung nach einem der vorhergehenden Ansprüche, **dadurch gekennzeichnet, dass** jeweils zwei oder mehrere der polymeren Filmbildner und anionischen Verdicker enthalten sind.

17. Verwendung nach einem der vorhergehenden Ansprüche, **dadurch gekennzeichnet, dass** zusätzlich UV-Filter, aktive Wirkstoffe zur Minderung von Hautfalten, Lösungsmittel, Feuchthaltemittel, Pflanzenextrakte, Vitamine, Antioxidantien, Emulgatoren, Tenside, Öle, Fette, Wachse, Silikone, Konservierungsmittel und/oder Parfümstoffe enthalten sind.
